# EUROPEAN PATENT APPLICATION

(11) **EP 1 935 422 A1**
(43) Date of publication of application: **25.06.2008**
(21) Application number: 06126705.0
(22) Date of filing: 20.12.2006
(51) Int. Cl.: A61K 31/455, A61K 9/16, A61K 9/20, A61P 13/12

(54) **Pharmaceutical composition comprising nicotinamide or nicotinic acid**

(71) Applicant: PEJO Iserlohn Heilmittel-und Diät-GmbH & Co.KG, 58638 Iserlohn (DE)
(72) Inventor: The designation of the inventor has not yet been filed
(74) Representative: Behnisch, Werner

(57) **Abstract**

The present invention is directed to a pharmaceutical composition for oral administration containing nicotinamide/nicotinic acid as an active ingredient, as well as to the use of this pharmaceutical composition for the treatment of hyperphosphatemia.

## Description

The present invention is directed to a pharmaceutical composition for oral administration containing nicotinamide/nicotinic acid as an active ingredient, as well as to the use of this pharmaceutical composition for the treatment of hyperphosphatemia.

### Background of the invention

Hyperphosphatemia is common in hemodialysis patients. Recent animal studies have shown that nicotinamide inhibits the sodium dependent phosphate co-transport in the small intestine and thereby reduces serum phosphorus levels (Eto et al., 2005). Nicotinic acid which is the prodrug of nicotinamide is widely used as antihyperlipidemic agent.

Katai et al. describe in "Nicotinamide inhibits sodium-dependent phosphate cotransport activity in rat small intestine, Nephrol Dial Transplant"; May 1999; 14(5): 1195-201, that the administration of niceritorol (a nicotinic acid derivative which improves lipid metabolism and peripheral circulation, and is used for the treatment of hyperlipidemia and impaired peripheral circulation) to patients with hyperphosphatemia undergoing dialysis decreased the serum phosphate (Pi) concentration. It was found out that this was due to an acceleration of faecal Pi excretion by niceritrol. It was suggested that nicotinamide may regulate the expression of a major functional Na/Pi cotransporter in the rat small intestine.

Takahashi et al. describe in "Nicotinamide suppresses hyperphosphatemia in hemodialysis patients"; Kidney Int. 2004 Mar; 65(3):1099-104 that the use of calcium- or aluminum-based phosphate binders against hyperphosphatemia is limited by the adverse effects of hypercalcemia or aluminum toxicity in long-term hemodialysis. Because nicotinamide is an inhibitor of sodium-dependent phosphate cotransport in rat renal tubule and small intestine, Takahashi et al. examined whether nicotinamide reduces serum levels of phosphorus and intact parathyroid hormone (iPTH) in patients undergoing hemodialysis. It was stated that nicotinamide may provide an alternative for controlling hyperphosphatemia and hyperparathyroidism without inducing hypercalcemia in hemodialysis patients.

Krishnaswamy Sampathkumar et al. ("Extended Release Nicotinic Acid - A Novel Oral Agent for Phosphate Control"; International Urology and Nephrology (2006) 38: 171-174) examined in a prospective study whether nicotinic acid reduces serum phosphorus levels in hemodialysis patients. A comparably small amount of nicotinic acid in a modified release form provided a sufficient efficacy for reducing serum phosphorus level.

The activity of nicotinamide for the treatment of hyperphosphatemia can be explained as follows:

In the intestinal wall, NaPi IIb co-transporter is influencing the phosphate uptake into the intestine. This co-transporter NaPi IIb is down-regulated by nicotinamide. If nicotinamide is subject to resorption through the intestinal wall, it is being lost for this effect of dow-regulation. If such a resorption occurs, nicotinamide may have an additional effect by influencing the NaPi IIa co-transporter in the kidney, whereby phosphate excretion by the kidney is increased. In this context, nicotinamide can positively influence the NaPi IIa co-transporter and thereby, phosphate excretion by the kidneys.

However, this effect can only be seen in patients, which still show a residual renal function (for example pre-dialytic patients). This effect cannot be achieved for dialytic patients, since due to a lack of renal function, the system of influencing the NaPi IIa co-transporter does not work anymore.

In animal trials performed by the inventors, it turned out that nicotinamide and nicotinic acid showed an equivalent *in vivo* behaviour in this regard.

As a summary, it is of utmost importance to provide a pharmaceutical formulation, which provides a uniform release of nicotinamide/nicotinic acid over time in order to ensure a uniform effect in particular on the NaPi IIb co-transporter in the wall of the intestine. For dialytic patients, this release should be sufficiently small in order to avoid resorption of nicotinamide/nicotinic acid in the intestine and, simultaneously, the NaPi IIb co-transporter into the intestine should be reliably down regulated. For this purpose, the amount of nicotinamide/nicotinic acid has to be provided in a sufficient, but not too large amount *in vivo.* The release of nicotinamide/nicotinic acid from the pharmaceutical formulation thus should be substantially linear.

### Summary of the invention

Therefore, it is a problem underlying the present invention to provide a pharmaceutical composition having a uniform and almost linear *in vivo* release pattern of nicotinamide/nicotinic acid. It is a further problem underlying the invention to provide a pharmaceutical composition, which is in particular suitable for treating hyperphosphatemia in dialytic and pre-dialytic patients.

The inventors made the attempt to influence the release behaviour of a nicotinamide formulation by coating the nicotinamide containing substrate with a commercially available pH independent coating. By this means, an immediate, complete release should be avoided, however, at the same time, nicotinamide should be provided in a suitable amount to the respective regions of the intestine by pH independent diffusion.

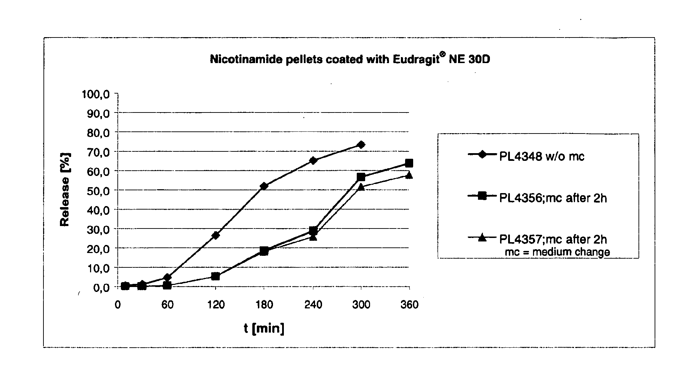

However, an examination of the release behaviour at a pH value of 1,2 (artificial gastric juice) surprisingly resulted in a release profile, which could not be expected for nicotinamide. It turned out that, in contrast to the scientific literature, nicotinamide is not released constantly at the different pH values, but unexpectedly showed a pH-dependent release pattern. In this connection, it is referred to Fig. 1 showing the unusual release pattern for coated nicotinamide pellets.

In the light of the above requirements for nicotinamide formulations for use in the therapy of hyperphosphatemia, the unmodified release pattern of nicotinamide is not sufficient. Since, as mentioned above, nicotinic acid is showing a very similar behaviour *in vivo,* this problem can also be extended to this substance.

The inventors found out that in order to overcome this problem, it is not suitable to provide only one dosage form showing a delayed or sustained release of nicotinamide/nicotinic acid, but additionally to provide a second dosage form, comprising an immediate release dosage of nicotinamide/nicotinic acid. By combining these two dosage forms, a more or less linear release behaviour of nicotinamide/nicotinic acid in vivo can be achieved, therefore, a product is provided which is specifically adapted to the treatment of hyperphosphatemia in particular in patients suffering from pre-dialysis or dialysis.

### Detailed description of the invention

According to a first aspect, a pharmaceutical composition for oral administration containing nicotinamide/nicotinic acid as an active ingredient is disclosed, comprising the following components:
a) a first dosage of said nicotinamide/nicotinic acid for immediate release of nicotinamide/nicotinic acid;
b) a second dosage of said nicotinamide/nicotinic acid and a means for delaying release of said second dosage of nicotinamide/nicotinic acid, wherein the means for delaying release are acting pH independently.

In the context of the present invention, the second dosage of nicotinamide/nicotinic acid can be regarded as the main component of formulation since it provides the body with the required basic levels of nicotinamide/nicotinic acid over time. However, in order to avoid the above mentioned drawbacks of the release behaviour of nicotinamide/nicotinic acid, the first dosage form is added in order to "smooth" the release curve for nicotinamide/nicotinic acid, i. e. making the curve more linear than it would be expected when using the second dosage alone (see Fig. 1).

According to a first embodiment, the composition comprises two distinct components, wherein the first component is containing the first dosage, the second component is containing the second dosage of nicotinamide/nicotinic acid. For example, both components can be present in spherical form, preferably in pellet form, separately comprising the first and second dosages of nicotinamide/nicotinic acid as indicated above. The first dosage form contains a first dosage of nicotinamide/nicotinic acid in an uncovered form, i. e. for immediate release of nicotinamide/nicotinic acid. The second component comprises as a means for delaying release a coating based on polymers which provide a non pH-independent release of said nicotinamide/nicotinic acid. Preferably, the polymers may be selected from Eudragit^{®} NE 30 D; Eudragit® RL 100/RS 100; Eudragit^{®} RL PO/RS PO; Eudragit^{®} RL 30 D/RS 30D or Eudragit^{®} 12,5/RS 12,5.

Further polymers, which may be used in this context are ethylcellulose, or commercially available products such as SURELEASE^{®} or AQUACOAT^{®} EC.

The following are preferred formulations, which may be used in the present invention. All formulations are provided with a pH independent coating.

| | |
|---|---|
| Eudragit® NE pellets: | nicotinamide |
| | microcrystalline cellulose |
| | Eudragit^{®} NE 30 D |
| | glycerine monostearate |
| | polysorbat 80 |

A similar release behaviour may be achieved by using Eudragit® RL and RS as well as mixtures thereof.

| | |
|---|---|
| Ethylcellulose pellets: | nicotinamide |
| | microcrystalline cellulose |
| | ethylcellulose* |
| | dibutyl sebacate* |
| | ammonium oleate* |

| | |
|---|---|
| *ingredients of SURELEASE^{®} (source: US patents 4,123,403 and 4,502,888). SURELEASE^{®} is a "ready to use"-aqueous suspension of Colorcon, PA, USA. A similar release behaviour can be achieved by using AQUACOAT^{®} ECD (aqueous formulation with ethylcellulose) of FMC BioPolymer, Pennsylvania, USA. | |

For further information, see also Pharmazeutische Technologie, Rudolf Voigt, Deutscher Apotheker Verlag Stuttgart; Pharmazeutische Technologie, Sucker/Fuchs/Speiser, Thieme Verlag; Pharmazeutische Technologie: Moderne Arzneiformen, Müller/Hildebrand, Wissenschaftliche Verlagsgesellschaft; Überzogene Arzneiformen, Bauer/Lehmann/ Osterwald/Rothgang, Wissenschaftliche Verlagsgesellschaft; each in its latest edition.

As a microtablet formulation, the following might be used:

| | |
|---|---|
| Microtablets: | nicotinamide |
| | microcrystalline cellulose |
| | magnesium stearate |
| | highly dispersed silica |

The microtablet may be formulated to a delayed release microtablet by using pH independent coating systems (as for the above pellets).

### Sachets:

Granules may be, for example, manufactured from nicotinamide, PVA and PVP, respectively, and by using the above coating systems for providing a delayed release effect.

As mentioned above, the first and the second component in the above embodiment is preferably present in spherical form, preferably in pellet form. However, also other pharmaceutical formulations as microtablets and the like are suitable for the first and second component of the pharmaceutical composition.

Regarding the second component, in the pharmaceutical art compositions are known which provide sustained release of pharmacologically active substances contained in the compositions after oral administration to humans and animals. Sustained release formulations known in the art include specifically coated pellets (as mentioned above), coated tablets and capsules and, furthermore, ion exchange resins, wherein the slow release of the active medicament is brought about through selective breakdown of the coating of the preparation or through compounding with a special matrix to effect the release of the drug.

For the first component (first dosage) it is in particular preferred that it is released during the retention time of the pharmaceutical composition in the stomach. In other words, the first dosage of nicotinamide/nicotinic acid has to be released during a very early stage shortly following administration of the pharmaceutical composition.

In a preferred embodiment, the first component comprises spheres, on which nicotinamide/nicotinic acid is coated. For example, the first component comprises spheres of pellets, on which a liquid containing nicotinamide/nicotinic acid is coated. The liquid is dried thereafter, leaving behind the dried psychostimulant on the pellets. Those pellets preferably are sugar pellets which act as carrier for the active substance nicotinamide/nicotinic acid. Those pellets preferably are consisting of a mixture of corn (maize), starch and sucrose.

The first and second components are (if they are formed as distinct components) preferably incorporated in a capsule, more preferably a hard gelatine capsule, or in a sachet.

In an alternative embodiment, the first and second dosages are contained in one single component. This one single component may be based on a multi-layer release technology, wherein a substrate (for example pellet) contains a series of layers having different characteristics. An outer immediate release layer comprising said first dosage of nicotinamide/nicotinic acid, an intermediate layer of pH independent coating material and an inner substrate containing the second dosage of nicotinamide/nicotinic acid (for example also present in the form of a pellet on which nicotinamide/nicotinic acid is coated) are envisioned. Many options can be chosen by a skilled person in order to carry out the solution of the present invention, i. e. to provide a substantially linear release of nicotinamide/nicotinic acid in vivo. Further information can be found, for example, in "Remmington's Pharmaceutical Sciences", Mack Publishing Corporation, Easton, PA, latest edition.

As mentioned above, the substrate material preferably contains an inert core material and most preferably is a sugar pellet.

The pharmaceutical composition of the present invention preferably has an overall content of nicotinamide/nicotinic acid (first and second dosage together) suitable to provide a daily dosage of from 50 to 2000 mg of nicotinamide/nicotinic acid. Very low dosages of down to 50 mg only may be suitable for predialysis patients. When nicotinamide/nicotinic acid is administered as sachets, the overall daily dosage may also be up to 1.500-2.000 mg per human patient.

This daily dosage may be provided in one single unit (having a corresponding content of nicotinamide/nicotinic acid) or by administering two or more distinct units of the pharmaceutical composition during the day. A pharmaceutical composition which is suitable for the latter purpose contains about 50 mg - 500 mg, preferably 200 mg - 400 mg nicotinamide/nicotinic acid. For example, 62,5 mg, 125 mg or 250 mg are envisioned as being a suitable single unit dosage which can be administered to a human patient.

In the present invention, it is preferred that the pharmaceutical composition comprises two distinct components wherein the first component is containing an immediate release dosage of nicotinamide/nicotinic acid, the second component containing a delayed release dosage. The background for this is, that both types of components usually are mixed in a combined dosage form (for example gelatine capsules) and two distinct components make it easier to set a precise ratio of the first dosage to the second dosage.

In this regard, the ratio of the first dosage to the second dosage (or the first component to the second component) preferably is 20:80 % by weight to 80:20 % by weight, more preferably 40:60 % by weight to 60:40 % by weight, most preferably 50:50 % by weight.

However, for specific purposes, the ratio of the first dosage to the second dosage may be adapted to different circumstances:

Therefore, according to a preferred embodiment, the first dosage is at least 50% of the overall content of nicotinamide/nicotinic acid in the composition. In other words, a comparably high amount of immediate release dosage is released from the pharmaceutical composition already during retention in the stomach. In this case, the ratio of the first dosage to the second dosage preferably is about 60:40 % by weight and the composition is intended for treating hyperphosphatemia in predialytic patients. In this case, a certain amount of nicotinamide/nicotinic acid is resorbed by the patient and influences the NaPi IIa co-transporter in the kidney in order to enhance phosphate excretion.

In a further embodiment, the amount of the first dosage to the second dosage is less than 50% by weight of the overall content of nicotinamide/nicotinic acid in the composition.

In this case, the ratio of the first to the second dosage preferably is about 40:60 % by weight and the composition is intended for treating hyperphosphatemia in dialytic patients. The background for this is that this precise composition will provide for a strictly linear release of nicotinamide/nicotinic acid and therefore, is in particular suitable for dialytic patients which do not possess any remaining renal function.

The pharmaceutical composition of the present invention preferably shows an *in vivo* release of the nicotinamide/nicotinic acid from that pharmaceutical composition such that nicotinamide/nicotinic acid is released in an essentially continuous rate over a time period of between 1-12 hours, preferably 3-8 hours, more preferably between 2-6 hours.

In a second aspect, the present invention is directed to the use of such a pharmaceutical composition for the manufacture of a medicament for the treatment of hyperphosphatemia. As mentioned above, depending from the precise composition, it can be used for treating hyperphosphatemia in predialytic patients or in dialytic patients.

The present invention now is described in more detail by the enclosed examples.

In figure 1, the release behaviour of Eudragit^{®} coated nicotinamide pellets are depicted. The pellets have been manufactured as indicated below. In the first example, no change of medium (medium = pH 6,8) occurs. In the second and third example, the medium used is pH 1,2 from time point 0-120 min., and pH 6,8 following 120 min.

The release behaviour is measured in a standard basket dissolution apparatus according to USP XXII at 100 rpm and at a temperature of 37 °C.

Nicotinamide pellets coated with Eudragit^{®} NE 30 D have been manufactured as follows:
Step 1: A wet mixture is manufactured of 70 % nicotinamide, 30 % microcrystalline cellulose and water using a Diosna-mixer, which is subsequently processed to pellets by extrusion. In a rounding device, the pellets are rounded to a spherical form.
Step 2: From 500 g pellets from step 1, delayed release pellets are manufactured in a Hüttlin Coating-apparatus (Unilab 05) by applying a lacquer suspension, consisting of 667 g Eudragit^{®} NE 30 D (pH-independent lacquer), 200 g talc (separation means), 1,4 g simethicone emulsion (anti-foaming agent) and 732 g of water. During the coating process, samples are taken following the application of 10 %, 15 %, 20 %, 25 %, 30 %, 35 % and 40 % (overall amount Eudragit^{®}) dry lacquer substance Eudragit^{®} NE, and a release pattern is established from these samples.

Profile with 15 % dry lacquer substance from Eudragit^{®} NE 30:
Step 1: see above
Step 2: From 500 g pellets from step 1, delayed release pellets are manufactured by applying a lacquer suspension consisting of 250 g Eudragit^{®} NE 30 D (pH-independent lacquer), 3,8 g glycerine monostearate (alternative separation means in place of talc), 1,5 g polysorbate 80 (emulsifying component) and 137 g water, using a Hüttlin Coating-apparatus (Unilab 05).

It is noted that the term "15 % of dry lacquer substance (LTS)" means: 75 g LTS of Eudragit^{®} NE 30 D in relation to 500 g pellets.

## Claims

1. A pharmaceutical composition for oral administration containing nicotinamide/nicotinic acid as an active ingredient, comprising the following components:
a) a first dosage of said nicotinamide/nicotinic acid for immediate release of nicotinamide/nicotinic acid;
b) a second dosage of said nicotinamide/nicotinic acid and a means for delaying release of said second dosage of nicotinamide/nicotinic acid, wherein the means for delaying release are acting pH independently.

2. The pharmaceutical composition of claim 1, wherein the composition comprises two distinct components, the first component containing the first dosage, the second component containing the second dosage of nicotinamide/nicotinic acid.

3. The pharmaceutical composition of claim 1 or 2, wherein the second component comprises as a means for delaying release a coating based on polymers which provide a non-pH independent release of said nicotinamide/nicotinic acid.

4. The pharmaceutical composition of claim 3, wherein the polymer is selected from Eudragit^{®} NE 30 D, Eudragit^{®} RL 100/RS 100; Eudragit^{®} RL PO/RS PO; Eudragit^{®} RL 30 D/RS 30D; Eudragit^{®} 12,5/RS12,5; Surelease^{®}, or Aquacoat^{®}ECD.

5. The pharmaceutical composition of one or more of the preceding claims, wherein the first and the second component is present in spherical form, preferably in pellet form.

6. The pharmaceutical composition of one or more of the preceding claims, wherein the first and the second component is present in the form of a microtablet.

7. The pharmaceutical composition of claim 5 or 6, wherein the first component comprises spheres on which nicotinamide/nicotinic acid is coated.

8. The pharmaceutical composition of one or more of the preceding claims, wherein the first and second components are incorporated in a capsule, preferably a hard gelatine capsule, or in a sachet.

9. The pharmaceutical composition of claim 1, wherein the first and second dosage are contained in one single component.

10. The pharmaceutical composition of claim 9, wherein the component comprises an inner substrate containing the second dosage of nicotinamide/nicotinic acid, an intermediate layer of a pH independent coating material, and an outer layer comprising said first dosage of nicotinamide/nicotinic acid.

11. The pharmaceutical composition of claim 10, wherein the substrate contains an inert core material, on which the second dosage of nicotinamide/nicotinic acid is coated.

12. The pharmaceutical composition of one or more of the preceding claims, wherein the overall content of the first and second dosage of nicotinamide/nicotinic acid is 100 mg - 500 mg, preferably 200 - 400 mg.

13. The pharmaceutical composition of one or more of the preceding claims, wherein the ratio of the first dosage to the second dosage is 20:80 % by weight to 80:20% by weight, preferably 40:60 % by weight to 60:40% by weight, most preferably 50:50% by weight.

14. The pharmaceutical composition of claim 13, wherein the amount of the first dosage is at least 50% of the overall content of nicotinamide/nicotinic acid in the composition.

15. The pharmaceutical composition of claim 14, wherein the ratio of the first dosage to the second dosage is about 60:40 % by weight and the composition is intended for treating hyperphosphatemia in predialytic patients.

16. The pharmaceutical composition of claim 13, wherein the amount of the first dosage is less than 50% by weight of the overall content of nicotinamide/nicotinic acid in the composition.

17. The pharmaceutical composition of claim 16, wherein the ratio of the first dosage to the second dosage is about 40:60 % by weight and the composition is intended for treating hyperphosphatemia in dialytic patients.

18. The pharmaceutical composition of one or more of the preceding claims, wherein the *in vivo* release of the nicotinamide/nicotinic acid from that pharmaceutical composition is such that nicotinamide/nicotinic acid is released in an essentially continuous rate over a time period of between 1-12 h, preferably 3-8 h, more preferably between 2-6 h.

19. The pharmaceutical composition of one or more of the preceding claims, wherein the first dosage of nicotinamide/nicotinic acid is released during the retention time of said pharmaceutical composition in the stomach.

20. Use of a pharmaceutical composition of one or more of the preceding claims for the manufacture of a medicament for the treatment of hyperphosphatemia.

21. The use of a composition of claims 14 or 15 for treating hyperphosphatemia in predialytic patients.

22. The use of a composition of claims 15 or 16 for treating hyperphosphatemia in dialytic patients.
